Europäisches Patentamt

European Patent Office

Office européen des brevets

(11) Publication number: **0 122 581**
**B1**

(12) EUROPEAN PATENT SPECIFICATION

(45) Date of publication of patent specification: **16.01.91**

(51) Int. Cl.⁵: **C 12 M 1/12, C 12 Q 1/24**

(21) Application number: **84103965.4**

(22) Date of filing: **09.04.84**

(54) Process for isolating bacteria in blood.

(30) Priority: **15.04.83 JP 65227/83**
**22.04.83 JP 70017/83**
**22.04.83 JP 70018/83**

(43) Date of publication of application:
**24.10.84 Bulletin 84/43**

(45) Publication of the grant of the patent:
**16.01.91 Bulletin 91/03**

(84) Designated Contracting States:
**DE FR GB NL SE**

(56) References cited:
**BE-A- 894 694**
**FR-A-1 138 452**
**US-A-2 672 431**
**US-A-4 164 449**
**US-A-4 410 630**

**JOURNAL OF CLINICAL MICROBIOLOGY, vol. 5,
no. 1, January 1977, pages 46-50, American
Society for Microbiology, US; C.H. ZIERDT et al.:
"Development of a lysis-filtration blood culture
technique"**

(73) Proprietor: **TERUMO KABUSHIKI KAISHA
trading as TERUMO CORPORATION
44-1, 2-chome, Hatagaya Shibuya-Ku
Tokyo 151 (JP)**

(72) Inventor: **Kaminagayoshi, Satoshi
5-25-2, Honmachi
Shibuya-ku Tokyo (JP)**

(74) Representative: **Henkel, Feiler, Hänzel & Partner
Möhlstrasse 37
D-8000 München 80 (DE)**

**Description**

The present invention relates to a process for the isolation of bacteria in blood.

Bacteria are present in blood of patients suffering from serious systemic infections such as septicemia or bacteremia. In the diagnosis of such systemic infections, blood tests for the identification of bacteria and antibiotics susceptibility are performed. In order to conduct such blood tests, isolation of bacteria from blood is required to be performed.

In conventional processes for the isolation from blood of bacteria, a colony is grown in the following manner. Sampled blood is mixed with a liquid culture medium, and culture is performed until the culture medium becomes turbid with propagated bacteria. Subsequently, the propagated bacteria are recovered and transplanted onto another culture medium such as a blood or chocolate agar medium.

In such conventional processes, it is difficult to directly isolate bacteria which is only present in blood in small numbers. These conventional processes require a pretreatment step of propagating bacteria before isolation of bacteria on an agar medium, resulting in complex and lengthy procedures. Bacteria propagation or multiplication in a medium as described above generally requires a propagation time of one to ten days, and colony growth requires a further one or two days.

When the propagated bacteria are transplanted to a isolator medium, the bacteria under testing may contaminate the surrounding atmosphere or other bacteria may be mixed thereinto.

From BE—A—894 694 there are known a pad assembly for use in a microorganism culture test which comprises, in sterile conditions, a water-absorbing pad which is impregnated with a culture medium for culturing given microorganisms and dried, a filter member bonded to the upper surface of said water absorbing pad and having submicron pores of a size capable of substantially preventing the microorganisms from passing therethrough, and a coating of an antibiotic deactivating agent applied to the upper surface of said filter member or to the common surface between said filter member and said water-absorbing pad, as well as testing apparatus having the pad assembly mounted in a vessel.

From US—A—4 164 449 there is known a method for concentrating and separating microbial pathogens present in a blood sammple. In carrying out said method initially, a blood sample is injected into a closed evacuated space within an elongated centrifugation receptacle which comprises a smooth continuous surface at one end thereof. The centrifugation article is then subjected to centrifugation causing any microbial pathogens present in the blood sample to move toward the end of the elongated centrifugation receptacle which comprises the smooth continuous surface and collect thereon. After centrifugation, the major portion of the residual blood sample is removed from the centrifugation receptacle and the separated and concentrated microbial pathogens can then be removed for quantitative and qualitative analysis.

It is an object of the present invention to provide a process for isolating blood bacteria, which allows direct sampling of the blood bacteria without requiring an additional process such as propagation of the blood bacteria, which allows isolation of the bacteria without transplantation into another culture medium, and in which a colony can be grown within a short period of time.

According to an aspect of the present invention, there is provided a process for isolating bacteria from blood and culturing the bacteria, comprising the steps of:

(i) mixing blood to be examined with a liquid containing a hemolytic reagent and an anticoagulant to obtain a mixture;

(ii) transferring the mixture to another vessel for performing a filtration of the mixture, said other vessel being provided with a filter having a water absorption member laminated thereon in contact with the lower surface of said filter, and said filter having a pore size sufficiently small that it blocks passage therethrough of the bacteria to be separated from the mixture and, at the sme time, allows a filtrate resulting from the filtering to be absorbed in said water absorption member laminated in contact with said lower surface of said filter; and

(iii) culturing the bacteria separated on the filter with the nutrients supplied from said water absorption member;

characterized in that the step (i) is performed by further adding a liquid culture medium, and in a liquid tightly sealed vessel.

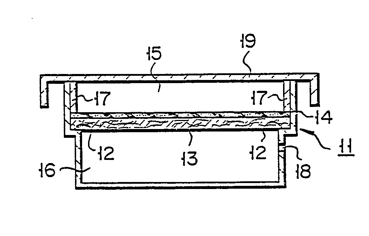

This invention can be more fully understood from the following detailed description when taken in conjunction with the accompanying Fig. 1 being a sectional view of an instrument for isolating bacteria from blood and culturing the isolated bacteria according to the process of the present invention.

The process of the present invention requires a step of homogeneously mixing sampled blood with a liquid containing a hemolysin and an anticoagulant and a liquid culture medium. This step is preferably performed in a sealed system so as to prevent both contamination of the surrounding atmosphere with the sampled bacteria and the introduction of foreign bacteria in the separated bacteria.

In order to prevent contamination of the blood by other bacteria, the mixing device is preferably sterilized by gamma ray radiation or autoclaving.

The blood treatment as described above damages red blood cells and also prevents blood coagulation, thereby allowing efficient subsequent blood filtration and blood bacterium culture.

After the blood treatment has been terminated the blood is removed from the mixing device by pouring onto a filter of an isolation and culturing instrument as described below.

Referring to the Fig., reference numeral 11 denotes a cylindrical vessel with a bottom and an open top. An annular step 12 is formed in the wall at a position intermediate along the direction of depth of the vessel. The periphery of a water absorption member 13 is supported on the upper surface of the step 12. A filter 14 having a pore size that does not allow passage of the bacteria to be cultured therethrough is placed on the upper surface of the member 13 throughout the entire open top of the vessel 11. Thus, the filter 14 integral with the water absorption member 13 divides the interior of the vessel 11 into an upper space 15 and a lower space 16. In order to prevent blood leakage into the lower space 16 through any gap between the wall of the vessel 11 and the periphery of the filter 14, a sealing member 17 is arranged to press down upon the periphery of the filter 14. Alternatively, the periphery of the filter 14 can be adhered to the wall of the vessel 11 with an adhesive.

A ventilation hole 18 for allowing the passage of air between the lower space 16 and the outer atmosphere is formed at an upper position of the wall of the vessel 11. Reference numeral 19 denotes a cover covering the open top of the vessel 11.

Any material inert to blood can be used as the material of the filter 14. Examples of such an inert material include nitrocellulose, polycarbonate, polyamide, cellulose ester and the like. Examples of such materials which are commercially available include "Millipore"® (product of Millipolar Corp.), "Metricell"® (product of German Instrument Company) and the like. The pore size of the filter 14 is determined in accordance with the application. However, the pore size of the filter 14 is generally 0.75 µm or less and preferably 0.45 µm, or less. The filter 14 is preferably treated to give good hydrophilic properties by a known method to provide good blood wettability.

The water absorption member 13 absorbs and holds therein the blood filtered through the filter 14 and thereby supplies the nutrients to the bacteria held on the filter 14. The water absorption member 13 preferably has a capacity to absorb the total amount of blood filtered through the filter 14. The material of the member 13 is preferably a filter paper of cellulose type, non-woven fabric or the like. The member 13 must be securely fixed either directly or through an adhesive to the rear surface of the filter 14. When the member 13 is not properly fixed to the filter 14, efficient filtering cannot be expected, and the supply of water and nutrients to the bacteria on the filter 14 becomes unsatisfactory. The adhesive to be applied between the filter 14 and the member 13 is conveniently a heat sealable adhesive of low-melting point polymeric fiber which does not impair filtration. The member 13 is impregnated with a liquid culture medium which is dried upon impregnation, as needed. The dried medium is dissolved in the filtered blood absorbed and held by the water absorption member 13, which supplies water and nutrients to the bacteria on the filter 14.

The upper space 15 above the filter 14 is for storing the sampled blood, and the lower space 16 is for storing the portion of blood which is not absorbed and held by the member 13.

The instrument having the construction described above is sterilized by gamma ray radiation or ethylene oxide gas so as to improve the reliability of the test.

Although the shape of the isolation and culturing instrument as usable in the present invention is not particularly limited, it is preferably circular. Neither is the size of the instrument limited. When 2 ml of blood are to be tested, the vessel main body should have a diameter of about 60 mm.

A process for isolating bacteria from blood according to the present invention using the instrument described above will now be described. A blood sample of the required amount is obtained and is then mixed with a liquid mixture containing a hemolytic reagent and an anticoagulant and a liquid culture medium. Saponin is preferably used as the hemolytic reagent. Sodium amylo sulfate or sodium polyanethol sulfonate is used as the anticoagulant. Any liquid culture medium which is generally used for propagating bacteria may be used.

The above process is a pretreatment to be followed by the filtration process and is performed so as to damage red blood cells and prevent blood coagulation. The blood treated in this manner is poured onto the filter 14 of the instrument and the cover 19 is closed. The blood is filtered by its own weight; the bacteria remain on the filter 14 while the blood is passed through the filter 14 and is absorbed by the water absorption member 13. Any excess blood which cannot be absorbed by the member 13 drips into the lower space 16 of the vessel. The air compressed by the filtered blood is exhausted through the ventilation hole 18, so that efficient blood filtering is performed. After filtration, the isolating and culturing instrument is kept at a constant temperature so as to grow a colony on the filter 14.

Since the vessel has a top opening of a size substantially the same as that of the filter, recovery of the bacteria is easy. After recovery of the bacteria, various bacterial tests such as identification or antibiotics susceptibility test can be performed.

As can be seen from the above description, when the process for isolating bacteria from blood is carried out, many advantages to be described below are obtained.

With conventional bacterial tests, propagation and culture of blood bacteria is first performed with a liquid culture medium. This process is lengthy. The process of the present invention does not require this step and can therefore shorten the overall test time. Furthermore, since the step of transplanting the bacteria from the propagation medium to the isolation medium is not required, the overall process is simple. Contamination of the surrounding atmosphere by the bacteria and introduction of foreign bacteria into the vessel which may be caused upon bacteria transplantation can thus be prevented.

When the process of the present invention is carried out, all the blood bacteria can be trapped and directly isolated so that the bacteria detection ratio is high and the number of bacteria in blood can be counted, if so desired.

Exampled

A membrane filter (manufactured by Toyo Roshi K.K.) of nitrocellulose having a pore size of 0.45 µm was used as the filter. A cellulose paper filter "NO—63F"® (Toyo Roshi K.K.) was used as a water absorption member. The filter and the water absorption member were adhered together by placing a low-melting point nylon® adhesive between the filter and the member and heat sealing. The filter and the water absorption member had a diameter of 50 mm and were assembled as shown in the Fig.

A comparison between this process and conventional processes was made. In the first conventional process, 2.0 ml of blood was poured into a vessel holding a culture medium, an anticoagulant and a hemolytic reagent (total amount: 1.0 ml). The resultant mixture was poured into an isolating instrument for culturing. In the second conventional process, liquid culture was performed with "Eiken No. 5"® (manufactured by Eiken K.K.). In the third conventional process, a "Vacutainer 50"® (manufactured by BD company) was used. The obtained result are shown in Table 1 below.

With *N. Meningitidis*, the conventional liquid culture medium process did not allow bacterium detection. However, the process of the present invention allowed detection and counting of the bacteria within one day. With the process of the present invention, since the bacteria are isolated in the form of a colony, identification and antibiotics susceptibility test could be performed immediately. The process of the present invention also provided better results than the conventional processes for other types of bacteria.

In Table 1 below, "+" denotes positive and "−" denotes negative.

Table 1

| Names of bacteria | Inoculated number of bacteria | After 1 day | | | After 2 days | | |
|---|---|---|---|---|---|---|---|
| | | Filter method | * Eiken No. 5 | ** Vacutainer 50 | Filter method | * Eiken No. 5 | ** Vacutainer 50 |
| *Neisseria meningit-idis* | 30 | 22 | – | – | 22 | – | – |
| | 22 | 17 | – | – | 17 | – | – |
| *Candida adbiceps* | 14 | 15 | – | – | 15 | – | + |
| | 2 | 3 | – | – | 3 | – | – |
| *Bacteroides fragilis* | 15 | 14 | – | + | 14 | + | + |
| | 4 | 7 | – | + | 7 | + | + |
| *Peptostrep-tococcus parvulus* | 17 | 0 | – | – | 18 | + | + |
| | 6 | 0 | – | – | 1 | + | + |

<u>Note:</u>

* ... A cultivation system for microorganisms in blood, which is developed by Eiken Kagaku Co. (Japan), and includes, in 45 ml of its aqueous solution, 0.3 g vegatable extract, 0.2 g yeast extract, 0.14 g meat extract, 0.45 g pepton, 0.14 g soy pepton, 0.45 g tryptone, 0.14 g grape sugar and etc.

** ... A cultivation system for microorganisms in blood, which is developed by Pecton-Dickinson & Co. (U.S.A.), and comprises a 50 ml tube accomodated with 45 ml of Peptone Broth 11 medium. The Peptone Broth 11 medium includes per one liter 20 g quadtone, 12 g gelatin, 5 g sodium chloride, 5 g dextrose, 2.2 g sodium bicarbonate and etc.

# EP 0 122 581 B1

## Claims

1. A process for isolating bacteria from blood and culturing the bacteria, comprising the steps of:

(i) mixing blood to be examined with a liquid containing a hemolytic reagent and an anticoagulant to obtain a mixture;

(ii) transferring the mixture to another vessel for performing a filtration of the mixture, said other vessel being provided with a filter having a water absorption member laminated thereon in contact with the lower surface of said filter, and said filter having a pore size sufficiently small that it blocks passage therethrough of the bacteria to be separated from the mixture and, at the same time, allows a filtrate resulting from the filtering to be absorbed in said water absorption member laminated in contact with said lower surface of said filter; and

(iii) culturing the bacteria separated on the filter with the nutrients supplied from said water absorption member;

characterized in that the step (i) is performed by further adding a liquid culture medium, and in a liquid tightly sealed vessel.

2. The process according to claim 1, characterized in that said water absorption member contains a dried culture medium.

## Patentansprüche

1. Verfahren zum Isolieren von Bakterien aus Blut und Züchten der Bakterien, umfassend folgende Stufen:

1. Vermischen des zu untersuchenden Bluts mit einer ein hämolytisches Mittel und ein Antikoagulationsmittel enthaltenden Flüssigkeit zur Gewinnung eines Gemischs;

2. Überführen des Gemischs in ein weiteres Gefäß zur Durchführung einer Filtration des Gemischs, wobei das weitere Gefäß mit einem Filter ausgestattet ist, auf dessen Unterseite ein Wasserabsorptionsteil aufkaschiert ist und dessen Porengröße ausreichend gering ist, um eine Passage der aus dem Gemisch abzutrennenden Bakterien (durch das Filter) zu blockieren und gleichzeitig eine Absorption des aus dem Filtrationsvorgang herrührenden Filtrats in dem auf die Unterseite des Filters aufkaschierten Wasserabsorptionsteil zum ermöglichen, und

3. Züchten der auf dem Filter abgetrennten Bakterien, wobei die Nährstoffe von dem Wasserabsorptionsteil zugeführt werden, dadurch gekennzeichnet, daß die Stufe 1) unter weiterer Zugabe eines flüssigen Kulturmediums in einem flüssigkeitsdicht versiegelten Gefäß durchgeführt wird.

2. Verfahren nach Anspruch 1, dadurch gekennzeichnet, daß das Wasserabsorptionsteil ein getrocknetes Kulturmedium enthält.

## Revendications

1. Procédé pour isoler des bactéries du sang et pour cultiver les bactéries, comportant les étapes suivantes:

(i) mélange du sang à examiner à un liquide contenant un réactif hémolytique et un anticoagulant afins d'obtenir un mélange;

(ii) transfert du mélange dans un autre récipient pour effectuer une filtration du mélange, ledit autre récipient étant muni d'un filtre ayant un élément d'absorption d'eau stratifié sur celui-ci en contact avec la surface inférieure dudit filtre, et ledit filtre ayant une taille de pores suffisamment petite pour bloquer le passage à travers eux des bactéries à séparer du mélange, et, en même temps, pour permettre à un filtrat résultant de la filtration d'être absorbé dans ledit élément d'absorption d'eau stratifié en contact avec ladite surface inférieure dudit filtre; et

(iii) culture des bactéries séparées sur le filtre avec les nutriments délivrés par ledit élément d'absorption d'eau;

caractérisé en ce que l'étape (i) est effectuée en ajoutant de plus un milieu de culture liquide, et ceci dans un récipient scellé de façon étanche vis-à-vis des liquides.

2. Procédé selon la revendication 1, caractérisé en ce que ledit élément d'absorption d'eau contient un milieu de culture séché.